# EUROPEAN PATENT APPLICATION

(11) **EP 4 700 780 A1**
(43) Date of publication of application: **25.02.2026**
(21) Application number: 25197756.7
(22) Date of filing: 22.08.2025
(51) Int. Cl.: G16H 10/40

(54) **SAMPLE ANALYSIS SYSTEM, INFORMATION MANAGEMENT SYSTEM AND MODULE, AND INFORMATION MANAGEMENT METHOD**

(30) Priority: 22.08.2024 CN 202411165564; 19.08.2025 CN 202511164075
(71) Applicant: Shenzhen Mindray Bio-Medical Electronics Co., Ltd., Shenzhen, Guangdong 518057 (CN)
(72) Inventor: JIANG, Xia, Shenzhen, 518057 (CN); WANG, Yani, Shenzhen, 518057 (CN); WANG, Beibei, Shenzhen, 518057 (CN); ZHANG, Ning, Shenzhen, 518057 (CN)
(74) Representative: KIPA AB

(57) **Abstract**

Embodiments of the present disclosure relates to a sample analysis system, an information management system and an information management method. A target information management interface is displayed on a display screen, so that the target information management interface displays information of a target object; a target object of interest on the displayed target information management interface is determined in response to a first interactive operation of a user; and quality control information based on PBRTQC related to the determined target object of interest is displayed on the display screen in response to a second interactive operation of a user. Therefore, the real-time quality control information based on PBRTQC can be embedded to the closed loop of processing real-time quality control abnormalities in medical laboratories.

## Description

### TECHNICAL FIELD

The present disclosure relates to the field of laboratory information management, in particular to a sample analysis system, an information management system, an information management module, a real-time quality control module and an information management method.

### BACKGROUND

During the process of clinical diagnosis and treatment of diseases, the quality of medical laboratory testing directly affects doctors' diagnosis and treatment of diseases. High-quality medical laboratory testing results rely on a sound quality control system in medical laboratories. Quality control in medical laboratories includes three aspects: pre-analytical, intra-analytical, and post-analytical quality control, and the intra-analytical quality control is the most critical process in the quality management system. The intra-analytical quality control includes internal quality control (IQC). At present, the common implementation of IQC involves laboratory personnel testing quality control products at a certain frequency, using statistical theory to calculate and evaluate the reliability of the test results, thereby observing and eliminating out-of-control factors in the testing process. However, there are some typical defects in IQC monitoring using quality control products: 1) the process of IQC monitoring using quality control products is not continuous, instead, it involves testing at single time points to estimate whether the analytical process is out of control; 2) quality control products are not patient samples, so there is a matrix effect, which affects IQC results; 3) implementing IQC with quality control products requires additional investment in quality control products, reagents, labor, and other costs, resulting in high overall expenses.

In recent years, patient-based real-time quality control (PBRTQC) may remedy some defects of current IQC due to its characteristics. The advantages of PBRTQC lie in that: 1) it can monitor the system in real-time using patient samples, enabling timely identification of an instrument in out-of-control status; 2) since patient samples are used, there is no influence of matrix effect; 3) PBRTQC is an analysis software, and does not require additional investment in quality control products, reagents, and labor, resulting in lower costs.

Therefore, some information technology companies have developed real-time quality control systems based on PBRTQC. However, the existing real-time quality control systems based on PBRTQC are not user-friendly for users when processing real-time quality control abnormalities in medical laboratories.

### SUMMARY

Based on this, the task of the present disclosure is to provide a sample analysis system, an information management system and an information management method, which can realize closed-loop processing of real-time quality control abnormalities in medical laboratories.

According to a first aspect of the present disclosure, a sample analysis system is provided, including:
at least one sample analyzer configured to detect a plurality of samples for at least one detection item, so as to obtain detection results of the plurality of samples for the at least one detection item, wherein the at least one sample analyzer is quality-controlled for the at least one detection item through at least one quality control product;
a display device including a display screen for displaying a target information management interface and for displaying quality control information based on PBRTQC (Patient-Based Real-Time Quality Control), wherein the target information management interface is configured to display information of a target object, and the target object includes at least one type of the sample analyzer(s), the samples, the detection item(s) and the quality control product(s), and the target information management interface is further configured for being capable of receiving a work instruction, so that at least one of the sample analyzer(s) can execute a corresponding operation based on the work instruction, wherein the quality control information based on PBRTQC is generated based on the detection results of multiple of the samples for at least one of the detection item(s), wherein each detection item that is quality-controlled through one of the quality control product(s) is associated with its respective quality control information based on PBRTQC; and
a controller communicatively connected with the display device and configured to:
control the display device to display the target information management interface on the display screen,
determine a target object of interest on the displayed target information management interface in response to a first interactive operation of a user, and
control the display device to display the quality control information based on PBRTQC on the display screen in response to a second interactive operation of the user, wherein the displayed quality control information based on PBRTQC at least includes the quality control information based on PBRTQC that is related to the determined target object of interest.

According to a second aspect of the present disclosure, an information management system is provided, including:
an information management module configured to generate a target information management interface for displaying information of a target object, wherein the target object includes at least one type of sample analyzer(s), samples, detection item(s) and quality control product(s), the sample analyzer(s) is(are) configured to detect the samples for the detection item(s), so as to obtain detection results of the samples for the detection item(s), and the sample analyzer(s) is(are) quality-controlled for the detection item(s) through the quality control product(s), wherein the target information management interface is further configured for being capable of receiving a work instruction, so that at least one of the sample analyzer(s) can execute a corresponding operation based on the work instruction;
a real-time quality control module configured to generate quality control information based on PBRTQC, wherein the quality control information based on PBRTQC is generated based on the detection results of multiple of the samples for at least one of the detection item(s), wherein each detection item that is quality-controlled through one of the quality control product(s) is associated with its respective quality control information based on PBRTQC; and
a control module configured to:
control the information management module to generate the target information management interface and output the target information management interface to a display screen, so as to display the target information management interface on the display screen,
determine a target object of interest on the displayed target information management interface in response to a first interactive operation of a user, and
control the real-time quality control module to generate the quality control information based on PBRTQC and output the quality control information based on PBRTQC to the display screen in response to a second interactive operation of the user, so as to display the quality control information based on PBRTQC on the display screen, wherein the displayed the quality control information based on PBRTQC at least includes the quality control information based on PBRTQC that is related to the determined target object of interest.

According to a third aspect of the present disclosure, an information management method is provided, including:
displaying a target information management interface on a display screen, so that the target information management interface displays information of a target object, the target object including at least one type of sample analyzer(s), samples, detection item(s) and quality control product(s), wherein the sample analyzer(s) is(are) configured to detect the samples for the detection item(s), so as to obtain detection results of the samples for the detection item(s), and the sample analyzer(s) is(are) quality-controlled for the detection item(s) through the quality control product(s), wherein the target information management interface is further configured for being capable of receiving a work instruction, so that at least one of the sample analyzer(s) can execute a corresponding operation based on the work instruction, wherein each detection item that is quality-controlled through one of the quality control product(s) is associated with its respective quality control information based on PBRTQC;
determining a target object of interest on the displayed target information management interface in response to a first interactive operation of a user, and
displaying quality control information based on PBRTQC related to the determined target object of interest on the display screen, in response to a second interactive operation of a user, wherein the quality control information based on PBRTQC is generated based on the detection results of multiple of the samples for at least one of the detection item(s).

In the technical solutions provided in the first to third aspects of the present disclosure, based on a user instruction, it is possible to directly jump from the currently displayed target information management interface to displaying the quality control information based on PBRTQC, which allows, for example, when a PBRTQC-based quality control abnormality is detected, the quality control information based on PBRTQC to be directly obtained , thereby facilitating a user to investigate the cause of the quality control abnormality through the quality control information based on PBRTQC.

### BRIEF DESCRIPTION OF THE DRAWINGS

Fig. 1 is a schematic block diagram of a sample analysis system according to some embodiments of the present disclosure;
Fig. 2 is a schematic view of a first embodiment of a target information management interface according to the present disclosure;
Fig. 3 is a schematic view of a second embodiment of a target information management interface according to the present disclosure;
Fig. 4 is a schematic view of a third embodiment of a target information management interface according to the present disclosure;
Fig. 5 is a schematic view of another state of the target information management interface of Fig. 2;
Fig. 6 is a schematic view of another state of the target information management interface of Fig. 3;
Fig. 7 is a schematic view of another state of the target information management interface of Fig. 4;
Fig. 8 is a schematic view showing a target information management interface and a real-time quality control interface according to some embodiments of the present disclosure;
Fig. 9 is a schematic view showing a target information management interface and a real-time quality control interface according to some other embodiments of the present disclosure;
Fig. 10 is a schematic view of a first embodiment of a real-time quality control interface according to the present disclosure;
Fig. 11 is a schematic block diagram of an information management system according to some embodiments of the present disclosure;
Fig. 12 is a schematic block diagram of a sample analysis system according to still some other embodiments of the present disclosure;
Fig. 13 is a schematic view of a second embodiment of a real-time quality control interface according to the present disclosure;
Fig. 14 is a schematic view of a fourth embodiment of a target information management interface according to the present disclosure;
Fig. 15 is a schematic view of a third embodiment of a real-time quality control interface according to the present disclosure; and
Fig. 16 is a schematic flowchart of an information management method according to some embodiments of the present disclosure.

### DETAILED DESCRIPTION

The technical solutions in the embodiments of the present disclosure will be explicitly and completely described below in conjunction with the accompanying drawings in the embodiments of the present disclosure. Apparently, the embodiments described are merely some of the embodiments of the present disclosure, rather than all of the embodiments. On the basis of the embodiments of the present disclosure, all the other embodiments obtained by those skilled in the art on the premise that no inventive effort is involved shall fall into the protection scope of the present disclosure.

As mentioned in the background, with the deepening of research and application of PBRTQC, more and more information technology companies have developed real-time quality control systems based on PBRTQC.

However, the real-time quality control systems based on PBRTQC in the related art are platforms based on sample result information and algorithms, and thus cannot completely realize closed-loop processing of sample-detection result-PBRTQC real-time quality control-sample-detection result. In other words, when a user finds that a quality control result based on PBRTQC is abnormal, the user still needs to find a corresponding information management software or system to complete subsequent abnormality processing.

Based on this, an embodiment of the present disclosure provides a technical solution, in which a real-time quality control system based on PBRTQC is added to the closed loop of processing real-time quality control abnormalities in medical laboratories. By using this technical solution, it is possible to improve convenience and efficiency of processing real-time quality control abnormalities by the user.

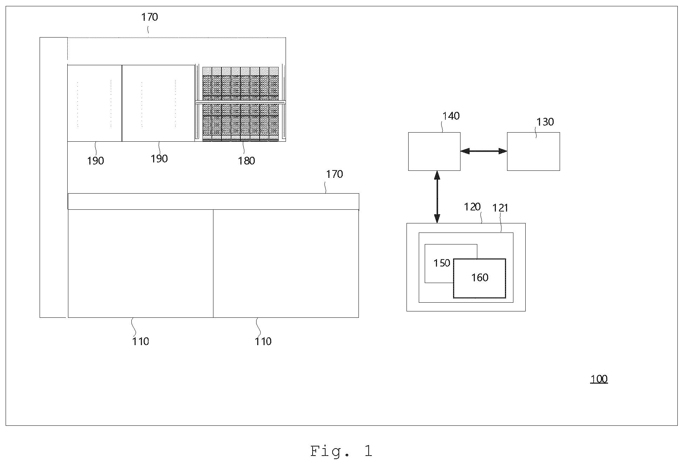

As shown in Fig. 1, an embodiment of the present disclosure provides a sample analysis system 100, which includes at least one sample analyzer 110, a display device 120 and a controller 140.

The at least one sample analyzer 110 is configured to detect a plurality of samples for at least one detection item, so as to obtain detection results of the plurality of samples for the at least one detection item. In addition, the at least one sample analyzer 110 is quality-controlled for the at least one detection item through at least one quality control product, that is, internal quality control (IQC), so as to determine whether the at least one sample analyzer is in control or out of control.

The display device 120 includes a display screen 121 for displaying a target information management interface 150, wherein the target information management interface 150 is configured to display information of a target object, and the target object includes at least one type of the sample analyzer(s), the samples, the detection item(s) and the quality control product(s). That is, the target information management interface may be a device information management interface for displaying information of the sample analyzer(s), or a sample information management interface for displaying information of the samples and the detection item(s) thereof, or a quality control product information management interface for displaying information of the quality control product(s). Here, the display screen 121 is further configured to display quality control information based on PBRTQC (Patient-Based Real-Time Quality Control), which is generated based on the detection results of multiple of the samples for at least one of the detection item(s). Preferably, the display screen 121 is configured to display a real-time quality control interface for displaying the quality control information based on PBRTQC.

In some embodiments, the target information management interface 150 may be further configured for being capable of receiving a work instruction, so that at least one of the sample analyzer(s) can execute a corresponding operation based on the work instruction.

In some embodiments, each detection item that is quality-controlled through one of the quality control product(s) is associated with its respective quality control information based on PBRTQC.

The controller 140 is communicatively connected with the display device 120 and configured to: control the display device 120 to display the target information management interface 150 on the display screen 121; determine a target object of interest on the displayed target information management interface 150 in response to a first interactive operation of a user (also referred to as a first user instruction), and control the display device 120 to display the quality control information based on PBRTQC on the display screen 121 in response to a second interactive operation of the user (also referred to as a second user instruction), wherein the displayed quality control information based on PBRTQC at least includes the quality control information based on PBRTQC that is related to the determined target object of interest (that is, the target object of interest indicated by the first interactive operation).

In some embodiments, the controller 140 configured to control the display device 120 to display the quality control information based on PBRTQC on the display screen 121 in response to a second interactive operation of a user, may include: the controller 140 configured to control the display device 120 to switch to displaying a real-time quality control interface 160, that is preferably independent of the target information management interface 150, on the display screen, or the controller configured to control the display device to display a real-time quality control interface, that is preferably independent of the target information management interface, on a same screen of the display screen, in response to the second interactive operation of the user, wherein the displayed real-time quality control interface 160 at least displays the quality control information based on PBRTQC that is related to the determined target object of interest.

In some other embodiments, the controller 140 configured to control the display device 120 to display the quality control information based on PBRTQC on the display screen 121 in response to a second interactive operation of a user, may include: the controller 140 configured to control the display device 120 to display, in a specific region on the target information management interface 150 displayed on the display screen 121, the quality control information based on PBRTQC that is related to the determined target object of interest.

In the embodiments of the present disclosure, by associating the target information management interface 150 with the quality control information based on PBRTQC, particularly the real-time quality control interface 160 based on PBRTQC, the quality control information based on PBRTQC, particularly the real-time quality control interface 160 can be directly invoked by the user for example via inputting an instruction on the target information management interface 150 without exiting from the current target information management interface 150 and manually opening the real-time quality control interface 160, which greatly facilitates the user to process a real-time quality control abnormality and improves the efficiency of processing the quality control abnormality.

For example, the sample analysis system 100 may further include an interactive device 130 configured to receive an interactive instruction from the user. Accordingly, the controller 140 may be configured to: receive a first user instruction of a user selecting a target object of interest from the displayed target information management interface 150; receive a second user instruction from the interactive device 130; and control the display device 120 to display the real-time quality control interface 160 based on PBRTQC on the display screen 121 in response to the second user instruction, so that the real-time quality control interface 160 displays at least real-time quality control information that is related to the target object of interest indicated by the first user instruction.

In some embodiments, the sample analyzer(s) 110 may be constructed as a biochemical analyzer, which may be configured to detect blood samples for various different detection items. The detection items include, for example, serum alkaline phosphatase (ALP) measurement, hepatitis B surface antigen (HBsAg) measurement, serum alanine aminotransferase (ALT) measurement, serum aspartate aminotransferase (AST) measurement, serum albumin (ALB) measurement and the like.

In some alternative or additional embodiments, the sample analyzer(s) 110 may be constructed as a coagulation analyzer, and the detection items include, for example, prothrombin time measurement, activated partial thromboplastin time measurement, thrombin time measurement, fibrinogen measurement and D-dimer measurement.

In other alternative or additional embodiments, the sample analyzer(s) 110 may be constructed as an immunoassay analyzer, and the detection items include, for example, tumor marker measurement, thyroid function measurement and the like.

In some embodiments, the at least one sample analyzer 110 may include a plurality of identical or different sample analyzers, for example, may include a biochemical analyzer, a coagulation analyzer and an immunoassay analyzer.

In some implementations, as shown in Fig. 1, the sample analysis system 100 may further include a dispatching device 170 for dispatching each of the samples to the at least one sample analyzer 110 for detection. Alternatively, the dispatching device 170 is also configured to dispatch the quality control product(s) to the at least one sample analyzer 110 for detection, so as to perform quality control analysis on the at least one sample analyzer 110.

In some embodiments, as shown in Fig. 1, the sample analysis system 100 may further include a sample management module 180 and a pre-processing module 190, wherein the sample management module 180 is configured to input or output a sample, and the pre-processing module 190 is configured to pre-process a sample or a sample container.

The sample management module 180 may also be referred to as a sample input and output device for placing a sample container carrying a sample. The sample input and output device is accessible by the user so as to receive a sample inputted by the user or allow the user to take away a sample. Here, the sample in the sample container may be a sample to be processed or a sample to be recovered.

As some implementations, the sample management module 180 may include a drawer, which is configured to place a sample carrying member and is movable between an open position where the drawer projects from a housing to facilitate an operator to retrieve and place a sample container and a closed position where the drawer is located inside the housing. In addition, the sample management module also includes a sample module manipulator, which is capable of transferring the sample container between the sample management module 180 and the dispatching device 170.

As some implementations, the pre-processing module 190 may include a centrifugal module and a decapping module, and a non-centrifuged sample may be centrifuged in the centrifugal module to realize the stratification of the blood sample. For example, the centrifugal module may include a housing and a centrifugal device, the centrifugal device is located in the housing, and a sample container is centrifuged in the centrifugal device. In addition, the centrifugal module may also include a centrifugal module manipulator, which is capable of transferring the sample container between the centrifugal module and a rail. The decapping module is configured to perform a decapping operation on a centrifuged sample container, so as to facilitate a suction needle of the sample analyzer 110 to subsequently extend into the sample container to suck the sample.

In some embodiments, as shown in Fig. 1, the dispatching device 170 is constructed as a transport rail including a main rail and a plurality of front-end rails connected to the main rail, wherein the vertical portion of the transport rail is the main rail and the horizontal portion of the transport rail is the front-end rail connected with the sample analyzer(s) 110, the sample management module 180, the pre-processing module 190 and the like.

In some embodiments, the display device 120 and the controller 140 may be integrated in a computer system directly or indirectly communicatively connected with the sample analyzer(s) 110.

In one example, the computer system may be a hospital laboratory information system capable of generating the target information management interface 150 for displaying information of the target object. In another example, the computer system may be a computer independent of the sample analyzer(s) 110, including a middleware loaded thereon that may is capable of generating the target information management interface 150 for displaying information of the target object.

In other embodiments, the display device 120 may be integrated in one of the sample analyzer(s) 110. Said sample analyzer integrated with the display device 120 is capable of generating the target information management interface 150 for displaying information of the target object. Alternatively, the controller 140 may also be integrated in said sample analyzer. In other words, the display device 120 and the controller 140 are a display device and a controller of one of the sample analyzer(s) 110.

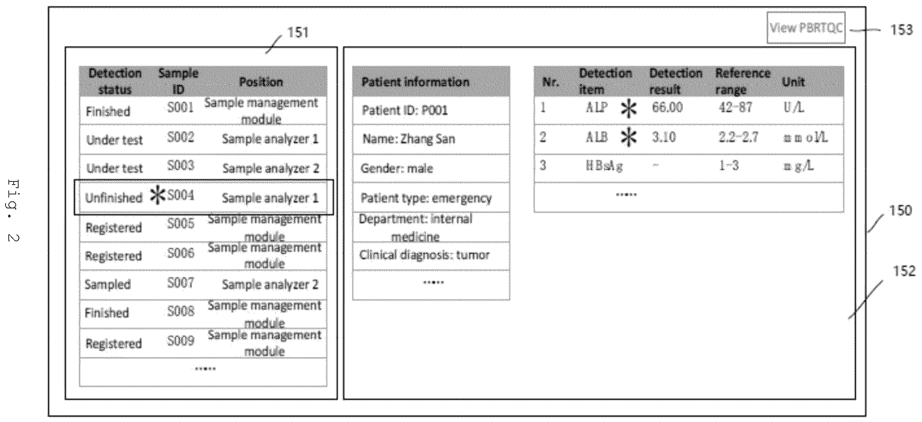

In some embodiments, as shown in Fig. 2, the target information management interface 150 may include a sample information management interface for displaying and managing information of the samples and the detection item(s) thereof (that is, for displaying the plurality of samples and sample information thereof as well as corresponding detection item(s) of each sample and the detection results thereof). Here, the target information management interface displays at least a sample list and a detection item list, for example. The sample list at least includes an ID, a detection state and a position in the system of each sample, and the detection item list at least includes each detection item and a detection result thereof.

For example, in the case where the target information management interface is the sample information management interface, the sample information management interface includes a first display area 151 and a second display area 152. The first display area 151 is configured to display a sample list of the samples, namely configured to display the plurality of samples and sample information thereof, and the second display area 152 is configured to display the detection item(s) of the sample selected in the sample list and the corresponding detection result(s) thereof, namely configured to display the detection item(s) corresponding to the sample selected from the plurality of samples displayed on the first display area and to display the detection results thereof. In the embodiment shown in Fig. 2, the second display area 152 displays the detection items of the sample S004 selected in the sample list of the first display area 151 and the corresponding detection results thereof.

In addition, the target information management interface 150 shown in Fig. 2 also displays patient information of the sample S004 selected in the sample list of the first display area 151.

In some embodiments, the sample information management interface may be further configured for being capable of receiving a detection instruction, which is one type of the work instruction, so that at least one of the sample analyzer(s) can detect at least one of the samples for at least one of the detection item(s) based on the detection instruction.

As some implementations, the sample information management interface may be configured to receive an instruction of re-detecting a sample, particularly an abnormal sample that is selected by the user on the sample information management interface. For example, the sample information management interface is provided with a virtual control, particularly a command button for dispatching a sample, so that the user may input a re-detection instruction by selecting the control. Accordingly, the controller 140 may be configured to control the dispatching device 170 to dispatch the selected sample to the corresponding sample analyzer for re-detection in response to the re-detection instruction.

As some other implementations, the sample information management interface may be configured to receive an instruction of dispatching a sample, particularly an abnormal sample selected on the sample information management interface to a user-accessible area, for example, to the sample management module 180.

Here, in the case where the target information management interface is the sample information management interface, the target object of interest is a sample of interest and/or a detection item of interest. Accordingly, the controller may be configured to determine a sample of interest and/or a detection item of interest on the displayed sample information management interface in response to the first interactive operation of the user, and control the display device to switch to displaying the real-time quality control interface on the display screen, or to display the real-time quality control interface on a same screen of the display screen, in response to the second interactive operation of the user, wherein the displayed real-time quality control interface at least displays the quality control information based on PBRTQC that is related to the determined sample of interest and/or detection item of interest.

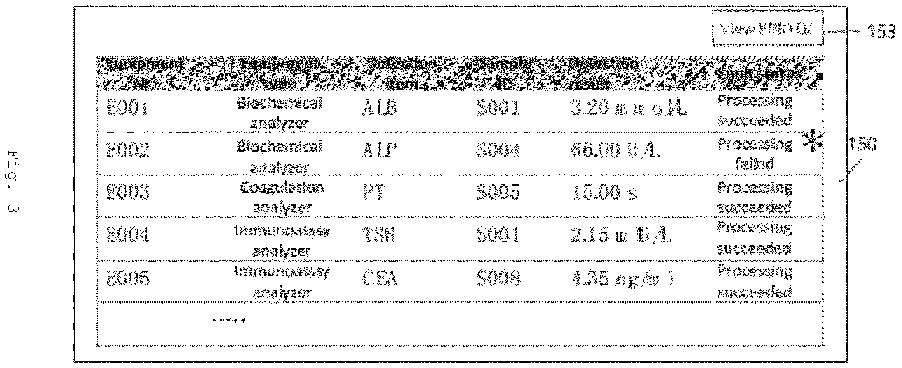

In some alternative or additional embodiments, as shown in Fig. 3, the target information management interface 150 may include a device information management interface for displaying and managing fault information of the sample analyzer(s). Here, the target information management interface displays, for example, at least a fault list, which at least includes information such as an equipment number, an equipment type, a detection item, a sample ID, a detection result and a fault status of the sample analyzer(s).

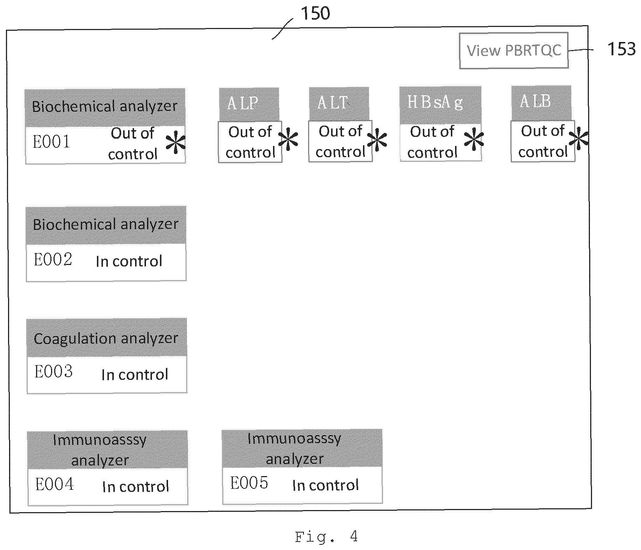

In some alternative or additional embodiments, as shown in Fig. 4, the target information management interface may include a quality control product information management interface for displaying and managing an analysis result of quality control analysis on the sample analyzer(s) using the quality control product(s)(that is, for displaying and managing display quality control information, which is obtained by performing quality control on at least one of the sample analyzer(s) for at least one of the detection item(s) through the quality control product(s)). Here, the target information management interface displays, for example, at least information such as an equipment type, an equipment number and an IQC quality control result (in control or out of control) of the sample analyzer(s).

In some embodiments, at least abnormal target object(s) is(are) displayed and preferably highlighted on the target information management interface 150, and the determined target object of interest is one of the abnormal target object(s). For example, in the case where the target information management interface 150 is the sample information management interface, abnormal sample(s) and/or abnormal detection item(s) is(are) highlighted on the sample information management interface, wherein the determined sample of interest is one of the abnormal sample(s) and/or the determined detection item of interest is one of the abnormal detection item(s).

In the embodiment shown in Fig. 2, the sample and the detection items with a quality control abnormality are highlighted by adding "*". In the embodiment shown in Fig. 3, the sample analyzer with a quality control abnormality is highlighted by adding "*". In the embodiment shown in Fig. 4, the sample analyzer and the detection items with an IQC quality control abnormality are highlighted by adding "*".

In some embodiments, the second interactive operation may include the user triggering a physical button for obtaining the quality control information based on PBRTQC, particularly for invoking the real-time quality control interface. For example, an interactive device 130 is provided to receive an interactive instruction from the user, and the controller 140 is configured to receive the second interactive operation from the interactive device 130. Here, the interactive device 130 may be constructed as a physical button that is signally connected with the controller 140.

In some other embodiments, the second interactive operation may include the user triggering a virtual button displayed on the target information management interface 150, for example on the sample information management interface for obtaining the quality control information based on PBRTQC, particularly for invoking the real-time quality control interface. For example, an interactive device 130 is provided to receive an interactive instruction from the user, and the controller 140 is configured to receive the second interactive operation from the interactive device 130. Here, the interactive device 130 may be a virtual control set on the target information management interface, and the controller 140 is configured to receive the second interactive operation of the user selecting the virtual control from the displayed target information management interface 150. For example, the virtual control is a virtual button. Of course, the embodiments of the present disclosure are not limited thereto, and the virtual control may also be an option in a menu bar or a control in other forms.

As shown in Figs. 2 to 4, the interactive device 130 is constructed as a virtual control 153 displayed on the target information management interface 150 for invoking the real-time quality control interface 160. Accordingly, the controller 140 configured to receive the second interactive operation from the interactive device, includes: the controller configured to receive the second interactive operation of the user selecting the virtual control 153 from the displayed target information management interface 150. That is, when the user selects the virtual control 153, for example, by clicking with a mouse, it is considered that the user has input the second interactive operation on the target information management interface 150.

In some embodiments, the controller 140 may be further configured to activate the virtual control 153 or the physical button in response to the first interactive operation. In other words, the virtual control 153 or the physical button is able to be selected or be operated to input the second interactive operation only in the case where the user inputs the first interactive operation, that is, in the case where the user selects the target object of interest. Otherwise, the virtual control 153 or the physical button is disabled.

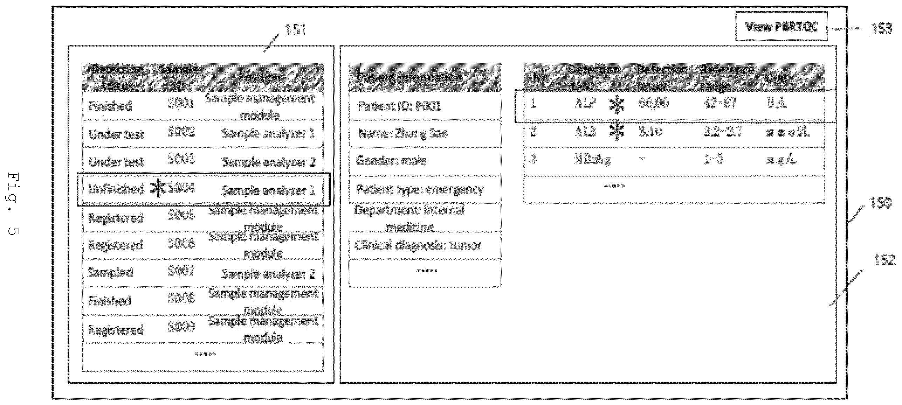

Fig. 5 shows the sample information management interface after the user selects a detection item of interest ALP on the sample information management interface shown in Fig. 2, wherein the virtual control 153 changes from an unavailable state shown in Fig. 2 to an activated state shown in Fig. 5.

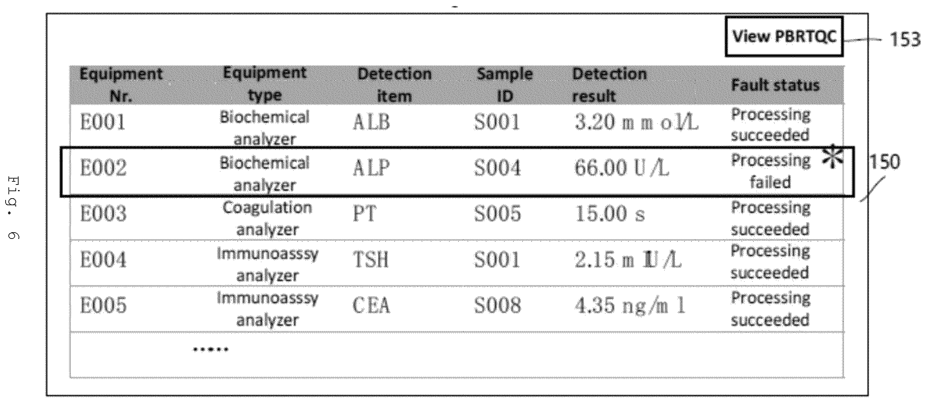

Fig. 6 shows the device information management interface in the case where the user selects the sample analyzer of interest E002 on the device information management interface shown in Fig. 3, wherein the virtual control 153 changes from an unavailable state shown in Fig. 3 to an activated state shown in Fig. 6.

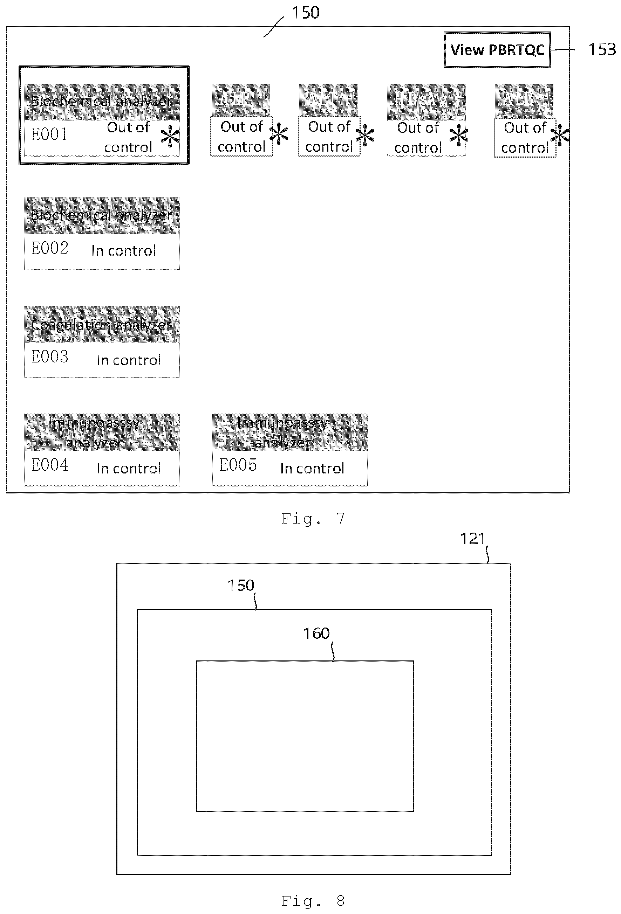

Fig. 7 shows the quality control product information management interface in the case where the user selects the sample analyzer of interest E001 on the quality control product information management interface shown in Fig. 4, wherein the virtual control 153 changes from an unavailable state shown in Fig. 7 to an activated state shown in Fig. 4.

In some embodiments, the controller 140 may be further configured to activate the virtual control 153 in response to the first interactive operation only when the determined target object of interest indicated by the first interactive operation is an abnormal target object.

In some embodiments, before jumping from the target object information management interface to the real-time quality control interface, it may be first determined whether the system supports and/or possesses real-time quality control based on PBRTQC. That is, the controller 140 may be further configured to: determine whether the sample analysis system 100 supports or possesses real-time quality control based on PBRTQC; and control the display device 120 to switch to displaying the real-time quality control interface 160 on the display screen 121 or to display the real-time quality control interface 160 in a same screen on the display screen in response to the second interactive operation if it is determined that the sample analysis system 100 supports or possesses the real-time quality control based on PBRTQC.

Further, the controller 140 may be further configured to: not control the display device 120 to perform any action in response to the second interactive operation if it is determined that the sample analysis system 100 does not support or possess the real-time quality control based on PBRTQC. That is, in the case where the sample analysis system 100 does not support or possess the real-time quality control based on PBRTQC, there is no response even if the second interactive operation is inputted.

Alternatively, the controller 140 may be further configured to: control the display device 120 to output a prompt on the display screen 121 in response to the second interactive operation if it is determined that the sample analysis system 100 does not support or possess the real-time quality control based on PBRTQC, so as to prompt the user that the system does not support viewing a real-time quality control condition based on PBRTQC.

Alternatively, the controller 140 may be further configured to: control the display device 120 to display the real-time quality control interface that is blank on the display screen 121 in response to the second interactive operation if it is determined that the sample analysis system 100 does not support or possess the real-time quality control based on PBRTQC.

Alternatively, the controller 140 may be further configured to: not activate the virtual control 153 if it is determined that the sample analysis system 100 does not support or possess the real-time quality control based on PBRTQC.

In some embodiments, the controller may be further configured to receive the first interactive operation inputted by a user from the displayed target information management interface 150 by one of the following methods: selecting a target object of interest in a target object list; selecting a target object of interest by searching or screening; and selecting a target object of interest by scanning a code.

For example, the first interactive operation may include one of the followings:
the user selecting the target object of interest from the target objects displayed on the target information management interface, preferably the user selecting the sample of interest from the samples displayed on the sample information management interface, and/or the user selecting the detection item of interest from the detection item(s) displayed on the sample information management interface;
the user selecting the target object of interest from the target objects displayed on the target information management interface by searching or screening, preferably the user selecting the sample of interest from the samples displayed on the sample information management interface, and/or the user selecting the detection item of interest from the detection item(s) displayed on the sample information management interface by searching or screening; and
the user selecting the target object of interest by scanning a code.

As some implementations, the user may directly perform a clicking operation on the displayed target information management interface 150 to input the first interactive operation, for example, clicking the target object of interest from the target object list.

In the embodiments shown in Figs. 2 and 5, the target information management interface is a sample information management interface for displaying the plurality of samples and sample information thereof as well as corresponding detection item(s) of each sample and the detection results thereof. The target information management interface 150 includes a first display area 151 and a second display area 152. The first display area is configured to display a sample list of the samples, namely to display the plurality of samples and sample information thereof, and the second display area is configured to display the detection items of the sample selected in the sample list and the corresponding detection results thereof, namely to display the detection item(s) corresponding to the sample selected from the plurality of samples displayed on the first display area and to display the detection results thereof. Here, the controller configured to receive a first user instruction of a user selecting a target object of interest from the displayed target information management interface, includes: the controller configured to receive the first user instruction of the user selecting a sample of interest from the first display area or the controller configured to receive the first user instruction of the user selecting a detection item of interest from the second display area. For example, the controller configured to determine a target object of interest (here is a sample of interest and/or a detection item of interest) on the displayed target information management interface (here is the sample information management interface) in response to a first interactive operation of a user, includes: the controller configured to determine a sample selected by the user from the plurality of samples displayed on the first display area, as the sample of interest, or to determine a detection item selected by the user from the detection item(s) displayed on the second display area, as the detection item of interest.

In the embodiment shown in Fig. 3, the target information management interface is a device information management interface for displaying and managing fault information of the sample analyzer(s), and the target information management interface displays at least a fault list. Here, the controller configured to receive a first user instruction of a user selecting a target object of interest from the displayed target information management interface, includes: the controller configured to receives the first user instruction of the user selecting a sample analyzer of interest from the fault list. For example, the controller configured to determine a target object of interest (here is a sample analyzer of interest) on the displayed target information management interface (here is the device information management interface) in response to a first interactive operation of a user, includes: the controller configured to determine a sample analyzer selected by the user from the fault list displayed on the device information management interface, as the sample analyzer of interest.

As some other implementations, the displayed target information management interface 150 is provided with a search control, so that the user may input a target object of interest in the search control to search the target object of interest.

As yet some other implementations, the displayed target information management interface 150 is provided with a screening control, so that the user may input an attribute of a target object of interest on the screening control so as to screen out the target object of interest.

As still some other implementations, the user may scan a sample bar code by a scanner that is signally connected with the display device, so as to input the target object of interest.

Some methods by which the controller 140 controls the display device 120 to display the quality control information based on PBRTQC, particularly the real-time quality control interface 160 on the display screen 121 will be described below in conjunction with some embodiments.

As some implementations, as shown in Fig. 8, the controller 140 may be configured to control the display device 120 to pop up a display frame that overlays the target information management interface 150, for example the sample information management interface on the display screen and to display the quality control information based on PBRTQC, particularly display the real-time quality control interface 160 in the display frame. Here, for example, the target information management interface 150 may be disabled, that is, it is no longer possible to perform user interaction on the target information management interface.

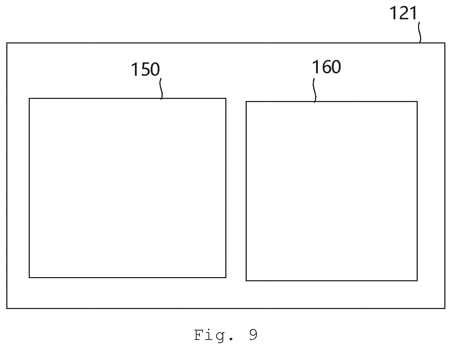

As some alternative implementations, as shown in Fig. 9, the controller 140 may be configured to control the display device 120 to display the target information management interface 150, for example the sample information management interface and the quality control information based on PBRTQC, particularly the real-time quality control interface 160 on a same screen in different display areas of the display screen.

As yet some other alternative implementations, the controller 140 may be configured to control the display device 120 to display the quality control information based on PBRTQC, particularly display the real-time quality control interface 160 on a tab on the display screen that is different from a tab where the target information management interface 150, for example the sample information management interface is displayed.

As still some other alternative implementations, the controller 140 may be configured to control the display device 120 to exit from the target information management interface 150, for example the sample information management interface and display the quality control information based on PBRTQC, particularly display the real-time quality control interface 160 on the display screen. It may be understood here that, exiting from the target information management interface 150 means that the target information management interface 150 is no longer displayed on the display screen, and does not mean that the target information management interface 150 no longer runs. It may be understood that, the target information management interface 150 may run in the background after exiting from the display screen.

Some implementations of the real-time quality control interface 160 will be described below in conjunction with some embodiments.

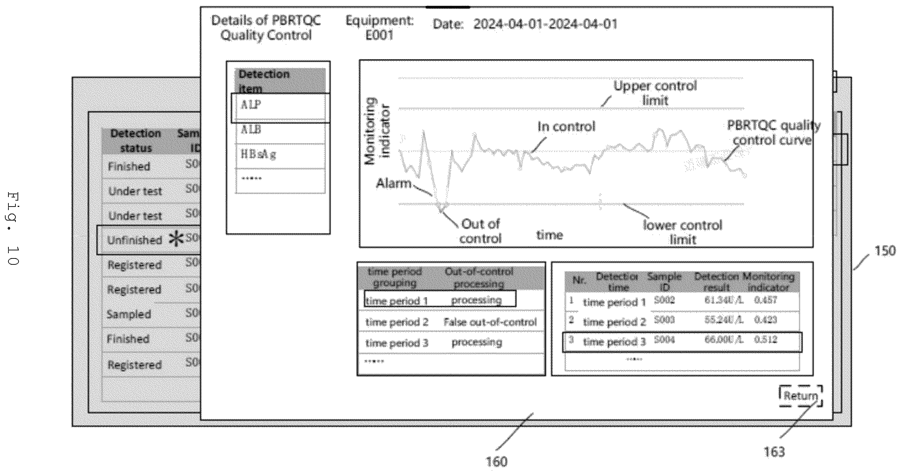

As some implementations, the quality control information based on PBRTQC may include a quality control curve based on PBRTQC. For example, the controller 140 may be further configured to control the display device 120 to display the real-time quality control interface 160 on the display screen 121, so that the real-time quality control interface displays at least a quality control curve based on PBRTQC that is related to the target object of interest indicated by the first interactive operation, as shown in Fig. 10.

In the embodiment shown in Fig. 10, the target object information management interface 150 is the sample information management interface shown in Fig. 2, wherein the user selects the detection item ALP of the sample S004 that is detected by the sample analyzer E001 on April 1, 2024 as the first interactive operation, and clicks the virtual control 153 to jump to the real-time quality control interface 160, so that the real-time quality control interface 160 displays the quality control curve based on PBRTQC that is related to the detection item ALP of the sample S004 that is detected by the sample analyzer E001 on April 1, 2024.

Further, the quality control information based on PBRTQC may further include abnormal sample(s) among the samples corresponding to the quality control curve based on PBRTQC, and grouping of the abnormal sample(s) as well as corresponding detection item(s) and the sample analyzer for detecting said samples. For example, the controller 140 may be further configured to control the display device 120 to display the real-time quality control interface 160 on the display screen 121, so that the real-time quality control interface also displays a grouping list of abnormal sample(s) among the samples for drawing the PBRTQC quality control curve, a sample list of the abnormal sample(s) belonging to corresponding groups, and the sample analyzer for detecting said samples.

For example, in the embodiment shown in Fig. 10, the real-time quality control interface also displays a time grouping list of abnormal samples among the samples for drawing the PBRTQC quality control curve, that is, groups with different time periods are displayed. In addition, the real-time quality control interface also displays a sample list of abnormal samples belonging to corresponding groups, for example, a sample list of abnormal samples belonging to a time period 1, and at least the detection time, the sample ID, the detection result and the monitoring indicator of each sample are listed in the sample list. In addition, the real-time quality control interface also displays the sample analyzer E001 for detecting each of the samples in the sample list.

Further, the abnormal sample(s) may be marked on the quality control curve based on PBRTQC. For example, the controller 140 may be further configured to mark, based on a user instruction, the abnormal sample(s) on the quality control curve based on PBRTQC, for example, highlight the monitoring indicator(s) of the abnormal sample(s).

In some embodiments, the controller 140 is further configured to perform one of the followings in response to a further user instruction or interactive operation: deleting or marking the quality control information based on PBRTQC for the abnormal sample(s); returning to the target information management interface; and switching to displaying a control product information management interface, wherein quality control product information management interface is configured to display quality control information, which is obtained by performing quality control on at least one of the sample analyzer(s) for at least one of the detection item(s) through the quality control product(s).

For example, the controller 140 may be configured to receive the further user instruction or interactive operation form the interactive device 130 or the real-time quality control interface 160.

As some implementations, the controller 140 may be further configured to delete or mark the quality control information based on PBRTQC for the abnormal sample(s) in response to a third interactive operation of the user (also referred to as a third user instruction).

For example, the controller 140 may be configured to receive the third interactive operation of deleting or marking the quality control information based on PBRTQC for the abnormal sample(s) from the real-time quality control interface 160. For example, when the user determines that the quality control information based on PBRTQC for the abnormal samples causing an out-of-control alarm indicates false out-of-control through analysis, the user can mark this out-of-control alarm as false out-of-control on the real-time quality control interface 160. For example, in the embodiment shown in Fig. 10, the out-of-control alarm of the time period 2 is marked as false out-of-control. After marking, the abnormal state identification related to this out-of-control alarm is eliminated synchronously in the sample analysis system.

It may be understood that, deleting the quality control information based on PBRTQC for the abnormal sample(s) does not mean permanent deletion, but that these deleted quality control information are no longer used in the real-time quality control based on PBRTQC.

As some other implementations, the controller 140 may be further configured to: in response to a fourth interactive operation of the user (also referred to as a fourth user instruction),
control the display device to switch back to displaying the target information management interface 150, for example the sample information management interface on the display screen; or
control the display device to switch to displaying a quality control product information management interface on the display screen, wherein quality control product information management interface is configured to display quality control information, which is obtained by performing quality control on at least one of the sample analyzer(s) for at least one of the detection item(s) through the quality control product(s).

In some examples, the fourth interactive operation is an instruction of returning to the target information management interface 150. For example, in the embodiment shown in Fig. 10, the real-time quality control interface 160 is provided with a virtual return control 163, preferably a command button, so that the user can input the fourth interactive operation by selecting the virtual return control 163.

Here, the controller is further configured to: receive a fifth interactive operation of the user (also referred to as a fifth user instruction) selecting a target object of interest from the real-time quality control interface 160; and control the display device 120 to display the target information management interface 150 on the display screen 121 in response to the fourth interactive operation, so that the target information management interface 150 displays at least information that is related to the target object of interest indicated by the fifth interactive operation.

For example, in the case where the target information management interface 150 is the sample information management interface, the controller 140 may be further configured to: before the controller controls the display device to switch back to displaying the target information management interface 150, namely the sample information management interface on the display screen, determine an abnormal sample of interest from the abnormal sample(s) displayed on the real-time quality control interface 160 and/or a detection item of interest corresponding to an abnormal sample in response to a fifth interactive operation of the user. Accordingly, the controller 140 configured to control the display device to switch back to displaying the target information management interface 150 on the display screen in response to a fourth interactive operation of the user, includes: the controller 140 configured to control the display device to switch back to displaying the sample information management interface on the display screen in response to the fourth interactive operation of the user, so that the sample information management interface at least displays information that is related to the abnormal sample of interest and/or the detection item of interest corresponding to the abnormal sample.

For example, in the embodiment shown in Fig. 10, if the fifth interactive operation is the user selecting the sample S004 on the real-time quality control interface, the controller 140 returns to the target information management interface 150 in response to the fourth interactive operation, and the target information management interface 150 displays at least the information that is related to the sample S004.

Further, as shown in Fig. 10, the controller configured to receive a fifth interactive operation of the user selecting a target object of interest from the real-time quality control interface, includes: the controller configured to receives the fifth interactive operation of the user selecting a target object of interest on the quality control curve based on PBRTQC or selecting a target object of interest in the grouping list or selecting a target object of interest in the sample list from the real-time quality control interface. For example, the fifth interactive operation may include: selecting the sample of interest from the quality control curve based on PBRTQC displayed on the real-time quality control interface, or selecting the sample of interest from the grouping displayed on the real-time quality control interface.

By returning to the target information management interface 150, particularly the sample information management interface, the user can apply for sample re-detection, sample dispatching, etc., through the target information management interface 150.

For example, when the user determines that the abnormal real-time quality control alarm based on PBRTQC indicates that there is a problem with the alarm through analysis, and this batch of corresponding abnormal samples related to the abnormal alarm need to be dispatched for re-detection, the user can directly jump to the sample information management interface through the real-time quality control interface, so as to complete the automatic dispatch and re-detection of said samples through the sample information management interface or to determine whether re-detection is required by the user.

In some other examples, the fourth interactive operation may be an instruction of switch to displaying the quality control product information management interface.

For example, when the user determines that the PBRTQC quality control of a batch of samples that have been detected by the sample analyzer E001 for the detection item ALP is abnormal through analysis, but cannot determine whether the quality control result of the sample analyzer E001 is really problematic. At this time, the user can directly jump to the quality control product information management interface through the real-time quality control interface 160, so as to dispatch a quality control product to conduct quality control analysis on the sample analyzer E001 for the detection item ALP through the quality control product information management interface. After IQC quality control is automatically performed, a corresponding quality control result will be fed back to the quality control product information management interface, so as to exclude the abnormal real-time quality control result.

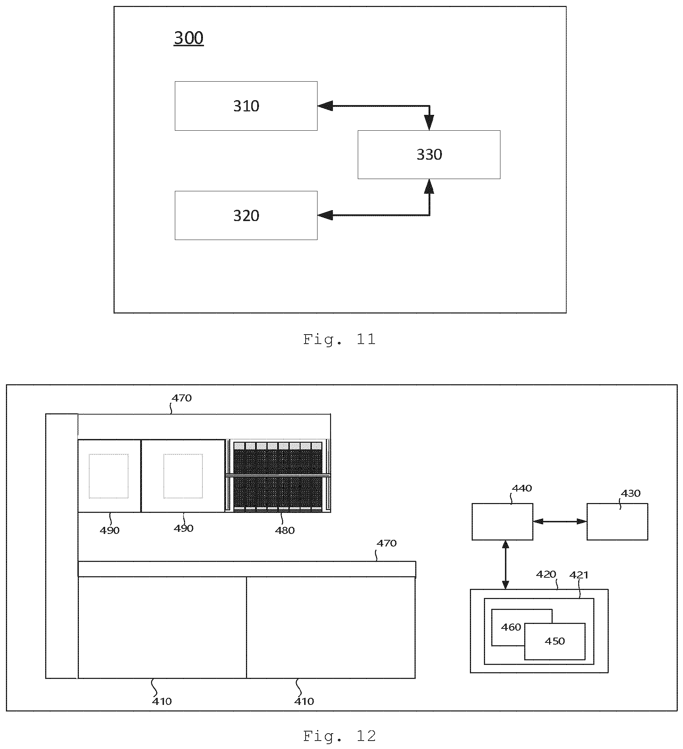

Fig. 11 shows a schematic block diagram of an information management system 300 according to some embodiments of the present disclosure.

As shown in Fig. 11, the information management system 300 includes an information management module 310, a real-time quality control module 320 based on PBRTQC and a control module 330.

The information management module 310 is configured to generate a target information management interface 150 for displaying information of a target object. Here, the target object includes at least one type of sample analyzer(s), samples, detection item(s) and quality control product(s), the sample analyzer(s) is(are) configured to detect the samples for the detection item(s), so as to obtain detection results of the samples for the detection item(s), and the sample analyzer(s) is quality-controlled analysis for the detection item(s) through the quality control product(s).

In some embodiments, the target information management interface 150 may be further configured for being capable of receiving a work instruction, so that at least one of the sample analyzer(s) can execute a corresponding operation based on the work instruction.

The real-time quality control module 320 is configured to generate quality control information based on PBRTQC, which is generated based on the detection results of multiple of the samples for at least one of the detection item(s).

In some embodiments, each detection item that is quality-controlled through one of the quality control product(s) is associated with its respective quality control information based on PBRTQC.

The control module 330 is configured to: control the information management module 310 to generate the target information management interface 150 and output the target information management interface to a display screen, so that the target information management interface is displayed on the display screen; determine a target object of interest on the displayed target information management interface in response to a first interactive operation of a user; and control the real-time quality control module 320 to generate the quality control information based on PBRTQC and output the quality control information based on PBRTQC to the display screen in response to a second interactive operation of the user, so that the quality control information based on PBRTQC is displayed on the display screen, wherein the displayed quality control information based on PBRTQC at least comprises the quality control information based on PBRTQC that is related to the determined target object of interest (that is, the target object of interest indicated by the first interactive operation).

Preferably, the real-time quality control module 320 is configured to generate a real-time quality control interface 160 for displaying the quality control information based on PBRTQC. Accordingly, the control module 330 configured to control the real-time quality control module 320 to generate the quality control information based on PBRTQC and output the quality control information based on PBRTQC to the display screen in response to a second interactive operation of a user, includes: the control module 330 configured to control the real-time quality control module 320 to generate the real-time quality control interface 160(preferably independent of the target information management interface) and output the real-time quality control interface to the display screen in response to the second interactive operation of the user, so as to switch to displaying the real-time quality control interface on the display screen, or to display the real-time quality control interface on a same screen of the display screen, wherein the displayed real-time quality control interface at least displays the quality control information based on PBRTQC that is related to the determined target object of interest.

For example, the control module 330 is configured to: control the information management module 320 to generate the target information management interface 150, so that the target information management interface displays information of the target object; receive an interactive instruction or interactive operation triggered by a user in the information management module 310; and control the real-time quality control module 160 to generate the real-time quality control interface 320 based on the interactive instruction.

For more embodiments of the target information management interface 150 and the real-time quality control interface 160, reference may be made to the above description of the sample analysis system 100 and its embodiments. That is, various embodiments of the target information management interface 150 and the real-time quality control interface 160 described above with reference to the sample analysis system 100 are also applicable to the target information management interface 150 generated by the information management module 320 and the real-time quality control interface 160 generated by the real-time quality control module 320.

In some embodiments, the information management system 300 may be implemented as software or hardware.

As some implementations, the information management system 300 is implemented as a software that may be loaded in a computer, for example, an application program, or implemented as a software that may run in a server (for example, a web server). Here, the information management module 310, the real-time quality control module 320 based on PBRTQC and the control module 330 are different software modules in the software.

As some other implementations, the information management module 310 and the real-time quality control module 320 based on PBRTQC are software that are different from each other and may be loaded in a computer or run in a server (for example, a web server). Here, the control module is, for example, a software module integrated in the information management module 310 or the real-time quality control module 320, or is an independent controller.

As yet some other implementations, the information management system 300 may also be implemented as a computer or a server, and the information management module 310, the real-time quality control module 320 based on PBRTQC and optionally the control module 330 may be installed in the computer or run in the server in the form of software.

As still some other implementations, the information management system 300 may also be implemented as a computer-readable storage medium, and the information management module 310, the real-time quality control module 320 based on PBRTQC and optionally the control module 330 are stored in the computer-readable storage medium in the form of software codes.

In some embodiments, the target information management interface 150 is a sample information management interface, which is configured to display the plurality of samples and sample information thereof as well as corresponding detection item(s) of each sample and the detection results thereof. In addition, the sample information management interface is further configured for being capable of receiving a detection instruction, which is one type of the work instruction, so that at least one of the sample analyzer(s) can detect at least of the samples for at least of the detection item(s) based on the detection instruction, wherein the target object of interest is a sample of interest and/or a detection item of interest.

In some embodiments, the second interactive operation may include the user triggering a physical button for obtaining the quality control information based on PBRTQC, particularly for invoking the real-time quality control interface.

In some other embodiments, the second interactive operation may include the user triggering a virtual button, for example a virtual button displayed on the target information management interface 150, for example the sample information management interface for obtaining the quality control information based on PBRTQC, particularly for invoking the real-time quality control interface.

In some embodiments, the control module 330 may be further configured to: control the information management module to generate the target information management interface, so that the target information management interface also displays a virtual control for invoking the real-time quality control interface; wherein the second interactive operation is an instruction of the user selecting the virtual control on the target information management interface. Of course, the embodiments of the present disclosure are not limited thereto, and the virtual control may also be an option in a menu bar or a control in other forms.

As shown in Figs. 2 to 4, the control module configured to receive an interactive instruction triggered by a user in the information management module 310, includes: the control module configured to receive the second interactive operation of the user selecting the virtual control 153 from the generated target information management interface 150. That is, when the user selects the virtual control 153, for example, by clicking with a mouse, it is considered that the user has input the second interactive operation on the target information management interface 150.

In some embodiments, the control module 330 may be further configured to activate the virtual control or the physical button in response to the first interactive operation. Preferably here, the virtual control is a command button. In other words, the virtual control 153 or the physical button is able to be selected or be operated to input the second interactive operation only in the case where the user inputs the first interactive operation, that is, where the user selects a target item of interest. Otherwise, the virtual control 153 or the physical button is disabled.

In some embodiments, the control module 330 may be further configured to control the information management module 310 to generate the target information management interface 150, so that at least abnormal target object(s) is(are) displayed and preferably highlighted on the target information management interface. Here, the determined target object of interest is one of the abnormal target object(s).

For example, in the case where the target information management interface is the sample information management interface, the control module 330 may be further configured to control the information management module to generate the sample information management interface, so that at least abnormal sample(s) and/or abnormal detection item(s) is(are) highlighted on the sample information management interface. Accordingly, the sample of interest is one of the abnormal sample(s) and/or the detection item of interest is one of the abnormal detection item(s).

In some embodiments, the control module 330 may be further configured to receive the first interactive operation inputted by the user from the target information management interface by one of the following methods: selecting a target object of interest in a target object list; selecting a target object of interest by searching or screening; and selecting a target object of interest by scanning a code.

For example, the first interactive operation may include one of the followings:
the user selecting the target object of interest from the target objects displayed on the target information management interface, preferably the user selecting the sample of interest from the samples displayed on the sample information management interface, and/or the user selecting the detection item of interest from the detection item(s) displayed on the sample information management interface;
the user selecting the target object of interest from the target objects displayed on the target information management interface by searching or screening, preferably the user selecting the sample of interest from the samples displayed on the sample information management interface, and/or the user selecting the detection item of interest from the detection item(s) displayed on the sample information management interface by searching or screening; and
the user selecting the target object of interest by scanning a code.

As some implementations, the user may directly perform a clicking operation on the displayed target information management interface 150 to input the first interactive operation, for example, clicking a target object of interest from the target object list.

In the embodiments shown in Figs. 2 and 5, the target information management interface is a sample information management interface for displaying information of the samples and the detection item(s) thereof. The target information management interface 150 includes a first display area 151 and a second display area 152. The first display area is configured to display a sample list of the samples, namely to display the plurality of samples and sample information thereof, and the second display area is configured to display the detection item(s) of the sample selected in the sample list and the corresponding detection result(s) thereof, namely to display the detection item(s) corresponding to the sample selected from the plurality of samples displayed on the first display area and to display the detection results thereof. Here, the control module may be configured to receive the first interactive operation of the user selecting a sample of interest from the first display area or receive the first interactive operation of the user selecting a detection item of interest from the second display area. For example, the control module configured to determine a target object of interest on the displayed target information management interface in response to a first interactive operation of a user, includes: the control module configured to determine a sample selected by the user from the plurality of samples displayed on the first display area, as the sample of interest, or to determine a detection item selected by the user from the detection item(s) displayed on the second display area, as the detection item of interest.

In the embodiment shown in Fig. 3, the target information management interface is a device information management interface for displaying and managing fault information of the sample analyzer(s), and the target information management interface displays at least a fault list. Here, the control module configured to receive a first interactive operation of the user selecting a target object of interest from the displayed target information management interface, includes: the control module configured to receive the first interactive operation of the user selecting a sample analyzer of interest from the fault list. For example, the control module configured to determine a target object of interest on the displayed target information management interface in response to a first interactive operation of a user, includes: the control module configured to determine a sample analyzer selected by the user from the fault list displayed on the device information management interface, as the sample analyzer of interest.

As some other implementations, the displayed target information management interface 150 is provided with a search control, so that the user can input a target object of interest on the search control to search the target object of interest.

As yet some other implementations, the displayed target information management interface 150 is provided with a screening control, so that the user can input an attribute of a target object of interest on the screening control so as to screen out the target object of interest.

As still some other implementations, the user may scan a sample bar code by a scanner that is signally connected with the display device, so as to input the target object of interest.

In some embodiments, the control module 330 may be further configured to control a display screen to display the target information management interface 150 and the quality control information based on PBRTQC, particularly the real-time quality control interface 160 by one of the following methods:
displaying the target information management interface, for example the sample information management interface on the display screen, and popping up a display frame that overlays the target information management interface, and displaying the quality control information based on PBRTQC, particularly the real-time quality control interface in the display frame, as shown in Fig. 8;
displaying the target information management interface, for example the sample information management interface and the quality control information based on PBRTQC, particularly the real-time quality control interface on a same screen in different display areas of the display screen, as shown in Fig. 9;
displaying the target information management interface, for example the sample information management interface in a first tab and displaying the quality control information based on PBRTQC, particularly the real-time quality control interface in a second tab that is different from the first tab on the display screen; and
displaying the target information management interface, for example the sample information management interface on the display screen, exiting from the target information management interface after generating the quality control information based on PBRTQC, particularly the real-time quality control interface and displaying the quality control information based on PBRTQC, particularly the real-time quality control interface on the display screen.

In some embodiments, the quality control information based on PBRTQC may include a quality control curve based on PBRTQC, and preferably the quality control information based on PBRTQC may further include abnormal sample(s) among the samples corresponding to the quality control curve based on PBRTQC, and grouping of the abnormal sample(s) as well as corresponding detection item(s) and the sample analyzer for detecting said samples, and further preferably the abnormal sample(s) may be marked on the quality control curve based on PBRTQC.

For example, as shown in Fig. 10, the control module 330 may be further configured to control the real-time quality control module 320 to generate the real-time quality control interface 160 in response to the second interactive operation, so that the real-time quality control interface displays at least a PBRTQC quality control curve that is related to the target object of interest indicated by the first interactive operation, and preferably so that, the real-time quality control interface also displays a grouping list of abnormal sample(s) among the samples for drawing the PBRTQC quality control curve, a sample list of the abnormal sample(s) belonging to corresponding groups and the sample analyzer for detecting said samples, and preferably so that the abnormal sample(s) is(are) marked on the PBRTQC quality control curve.

In some embodiments, the control module 330 may be further configured to perform one of the followings in response to a further user instruction or interactive operation: deleting or marking the quality control information based on PBRTQC for the abnormal sample(s); returning to the target information management interface; and switching to displaying a control product information management interface, wherein quality control product information management interface is configured to display quality control information, which is obtained by performing quality control on at least one of the sample analyzer(s) for at least one of the detection item(s) through the quality control product(s).

For example, the control module 330 may be configured to receive the further user instruction or interactive operation form the interactive device or the real-time quality control interface.

As some implementations, the control module 330 may be further configured to delete or mark the quality control information based on PBRTQC for the abnormal sample(s) in response to a third interactive operation of the user.

As some other alternative and additional implementations, the control module 330 may be further configured to: in response to a fourth interactive operation of the user,
control the information management module 310 to output the target information management interface, for example the sample information management interface to the display screen, so as to switch back to displaying the target information management interface on the display screen; or
control the information management module 310 to generate a quality control product information management interface and output the same to the display screen, so as to switch to displaying the quality control product information management interface on the display screen, wherein quality control product information management interface is configured to display quality control information, which is obtained by performing quality control on at least one of the sample analyzer(s) for at least one of the detection item(s) through the quality control product(s).

Further, the fourth interactive operation may be an instruction of returning to the target information management interface; and the control module 330 may be further configured to: receive a fifth interactive operation of the user selecting a target object of interest from the real-time quality control interface; and control the information management module to generate the target information management interface in response to the fourth interactive operation, so that the target information management interface displays at least information that is related to the target object of interest indicated by the fifth interactive operation.

For example, in the case where the target information management interface 150 is the sample information management interface, the control module 330 may be further configured to: before the control module controls the information management module to output the target information management interface to the display screen, so as to switch back to displaying the target information management interface on the display screen, determine an abnormal sample of interest from the abnormal sample(s) displayed on the real-time quality control interface and/or a detection item of interest corresponding to an abnormal sample in response to a fifth interactive operation of the user. Accordingly, the control module 330 configured to control the information management module to output the target information management interface to the display screen, so as to switch back to displaying the target information management interface on the display screen, in response to a fourth interactive operation of the user, includes: the control module 330 configured to control the information management module to output the sample information management interface to the display screen in response to the fourth interactive operation of the user, so as to switch back to displaying the sample information management interface on the display screen, so that the sample information management interface at least displays information related to the abnormal sample of interest and/or the detection item of interest corresponding to the abnormal sample.

Further, the quality control information based on PBRTQC may include a quality control curve based on PBRTQC, and the quality control information based on PBRTQC may further include abnormal sample(s) among the samples corresponding to the quality control curve based on PBRTQC, and grouping of the abnormal sample(s) as well as corresponding detection item(s). Here, the fifth interactive operation may include: selecting the sample of interest from the quality control curve based on PBRTQC displayed on the real-time quality control interface, or selecting the sample of interest from the grouping displayed on the real-time quality control interface.

For example, the control module 330 may be further configured to control the real-time quality control module to generate the real-time quality control interface in response to the second interactive operation, so that the real-time quality control interface displays at least a PBRTQC quality control curve that is related to the target object of interest indicated by the first interactive operation, a grouping list of abnormal sample(s) among the samples for drawing the PBRTQC quality control curve, and a sample list of the abnormal sample(s) belonging to corresponding groups; and the control module 330 configured to receive a fifth interactive operation of the user selecting a target object of interest from the real-time quality control interface, includes: the control module configured to receives the fourth fifth interactive operation of the user selecting a target object of interest on the PBRTQC quality control curve or selecting a target object of interest in the grouping list or selecting a target object of interest in the sample list from the real-time quality control interface.

It should be understood that, various embodiments described in connection with the sample analysis system 100, for example, the embodiments regarding the steps performed by the controller 140, are also applicable to the information management system 300.

As shown in Fig. 12, an embodiment of the present disclosure further relates to another sample analysis system 400, which includes at least one sample analyzer 410, an interactive device 430 and a controller 440.

The at least one sample analyzer 410 is configured to detect a plurality of samples for at least one detection item, so as to obtain detection results of the plurality of samples for the at least one detection item. In addition, the at least one sample analyzer 410 is quality-controlled for the at least one detection item through at least one quality control product, so as to determine whether the sample analyzer is under control or out of control.

The display device 420 includes a display screen 421 for displaying a real-time quality control interface 460 based on PBRTQC, wherein the real-time quality control interface is configured to display quality control information based on PBRTQC, which is generated based on the detection results of multiple of the samples for at least one of the detection item(s). In addition, the display screen 421 is further configured to display a target information management interface for displaying information of a target object. Here, and the target object includes at least one type of the sample analyzer(s), the samples, the detection item(s) and the quality control product(s).

The controller 440 is communicatively connected with the display device 420 and configured to: control the display device to display the real-time quality control interface on the display screen; determine a target object of interest on the real-time quality control interface in response to a fifth interactive operation of a user; and control the display device to switch to displaying the target information management interface on the display screen, or control the display device to display the target information management interface on a same screen of the display screen, in response to a fourth interactive operation of the user, wherein the displayed target information management interface at least displays information that is related to the determined target object of interest (that is, the target object of interest indicated by the fifth interactive operation).

In the embodiment of the present disclosure, by associating the target information management interface 450 with the real-time quality control interface 460 based on PBRTQC, the target information management interface 450 can be directly invoked by the user via inputting an instruction on the real-time quality control interface 460 without exiting from the current real-time quality control interface and manually opening the target information management interface, which greatly facilitates the user to process abnormal real-time quality control and improves the efficiency of processing the abnormal quality control.

In some embodiments, the sample analysis system 400 may further includes an interactive device 430 configured to receive an interactive instruction of the user. Accordingly, the controller 440 is communicatively connected with the display device 420 and the interactive device 430 and configured to: receive a fifth interactive operation of the user selecting a target object of interest from the real-time quality control interface 460; receive a fourth interactive operation inputted by a user from the interactive device 430, and control the display device 420 to switch to displaying the target information management interface on the display screen, or control the display device to display the target information management interface on a same screen of the display screen, in response to the fourth interactive operation, wherein the displayed target information management interface at least displays information that is related to the target object of interest indicated by the fifth interactive operation).

In some implementations, as shown in Fig. 12, the sample analysis system 400 may further include a dispatching device 470 for dispatching each of the samples to the at least one sample analyzer 410 for detection. Alternatively, the dispatching device 470 is also configured to dispatch the quality control product(s) to the at least one sample analyzer 410 for detection, so as to perform quality control analysis on the at least one sample analyzer 410.

In some embodiments, as shown in Fig. 12, the sample analysis system 400 may further include a sample management module 480 and a pre-processing module 490, wherein the sample management module 480 is configured to input or output a sample, and the pre-processing module 490 is configured to pre-process a sample or a sample container.

The sample management module 480 may be referred to as a sample input and output device for placing a sample container carrying a sample. The sample input and output device is accessible by the user so as to receive a sample inputted by the user or allow the user to take away a sample. Here, the sample in the sample container may be a sample to be processed or a sample to be recovered.

It should be understood that, various embodiments of the sample analyzer 110, the dispatching device 170, the sample management module 180 and the pre-processing module 190 described in connection with the sample analysis system 100 are also applicable to the sample analyzer 410, the dispatching device 470, the sample management module 480 and the pre-processing module 490 of the sample analysis system 400.

In some embodiments, the display device 420 and the controller 440 may be integrated in a computer system directly or indirectly communicatively connected with the sample analyzer(s) 410.

In one example, the computer system may be a hospital laboratory information system. In another example, the computer system may be a computer independent of the sample analyzer(s) 410, including a middleware loaded thereon.

In some other embodiments, the display device 420 may be integrated in one of the sample analyzer(s) 410. Alternatively, the controller 440 may also be integrated in said sample analyzer.

In some embodiments, the target information management interface 450 may be a device information management interface for displaying information of the sample analyzer(s), or a sample information management interface for displaying information of the samples and the detection item(s) thereof, or a quality control product information management interface for displaying information of the quality control product(s).

In some embodiments, the interactive device 430 may be constructed as a physical button signally connected with the controller 440, for example.

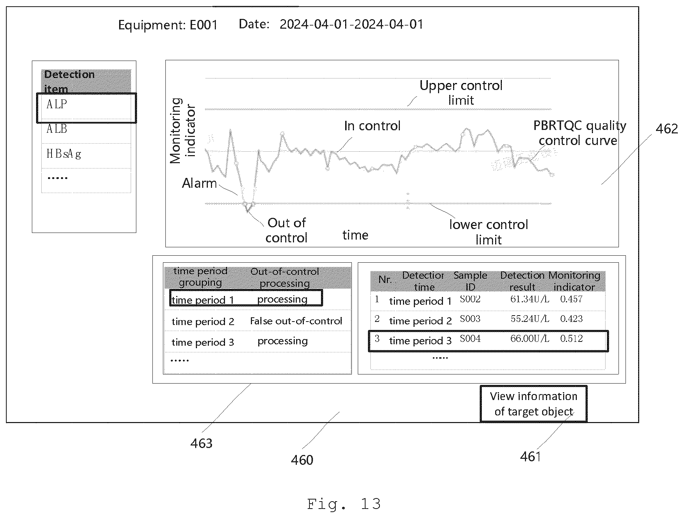

In some other embodiments, as shown in Fig. 13, the interactive device 430 may also be a virtual control, for example a virtual button set on the real-time quality control interface 460. For example, the virtual control is a command button. Of course, the embodiments of the present disclosure are not limited thereto, and the virtual control may also be an option in a menu bar or a control in other forms.

As some implementations, as shown in Fig. 13, the quality control information displayed on the real-time quality control interface at least includes a quality control curve based on PBRTQC, for example, the real-time quality control interface 460 displays at least a quality control curve based on PBRTQC that is related to the target object of interest indicated by the fifth interactive operation. For example, in the embodiment shown in Fig. 13, if the fifth interactive operation is the user selecting the sample S004 on the real-time quality control interface, the real-time quality control interface 460 displays at least a quality control curve based on PBRTQC related to the sample S004.

Further, the real-time quality control interface 460 also displays a grouping list of abnormal sample(s) among the samples for drawing the PBRTQC quality control curve, a sample list of the abnormal sample(s) belonging to corresponding groups, and the sample analyzer for detecting said samples. In other words, the real-time quality control interface further displays abnormal sample(s) among the samples corresponding to the quality control curve based on PBRTQC, and grouping of the abnormal sample(s) as well as corresponding detection item(s).

For example, in the embodiment shown in Fig. 13, the real-time quality control interface also displays a time grouping list of abnormal sample(s) among the samples for drawing the PBRTQC quality control curve, that is, groups with different time periods are displayed. In addition, the real-time quality control interface also displays a sample list of the abnormal sample(s) belonging to corresponding groups, for example, a sample list of the abnormal sample(s) belonging to a time period 1, so that at least the detection time, the sample ID, the detection result and the monitoring indicator of the samples are listed in the sample list. In addition, the real-time quality control interface also displays the sample analyzer E001 for detecting each of the samples in the sample list.

Further, the controller 440 may be further configured to mark the abnormal sample(s) on the PBRTQC quality control curve based on a user instruction, for example, highlight the monitoring indicator(s) of the abnormal sample(s).

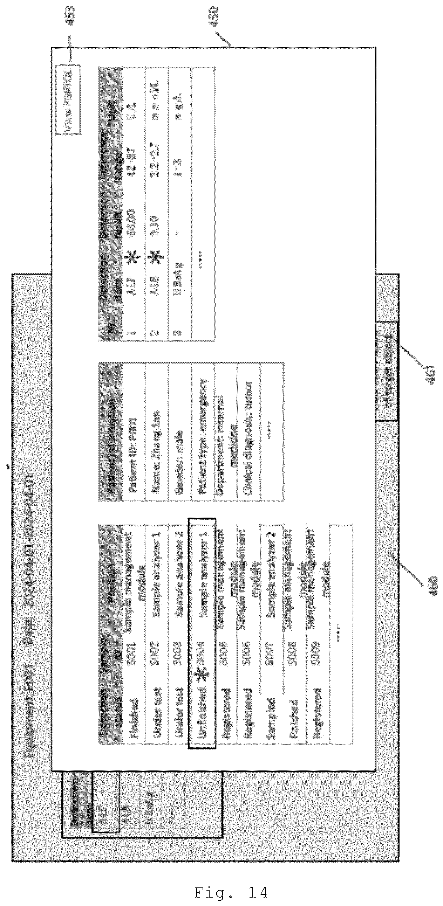

For example, in the embodiments shown in Figs. 13 and 14, if the fifth interactive operation is the user selecting the sample S004 on the real-time quality control interface, the controller 440 controls the display device to display the target information management interface 450 on the display screen in response to the fourth interactive operation, so that the target information management interface 550 displays at least the information related to the sample S004.

Further, as shown in Fig. 13, the controller configured to receive a fifth interactive operation of the user selecting a target object of interest from the real-time quality control interface, includes: the controller configured to receive the fifth interactive operation of the user selecting a target object of interest on the PBRTQC quality control curve or selecting a target object of interest in the grouping list or selecting a target object of interest in the sample list from the real-time quality control interface. For example, the fifth interactive operation may include: selecting the sample of interest from the quality control curve based on PBRTQC displayed on the real-time quality control interface, or selecting the sample of interest from the grouping displayed on the real-time quality control interface.

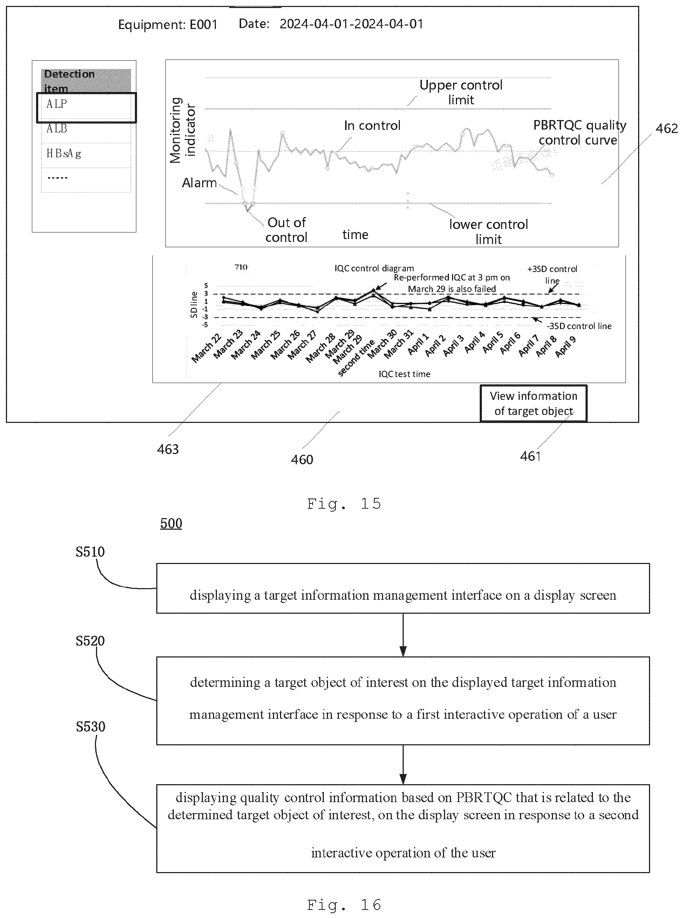

In some embodiments, the real-time quality control interface 460 displays the quality control curve based on PBRTQC that is related to the target object of interest indicated by the fifth interactive operation in the third display area 462, displays abnormal sample(s) among the samples corresponding to the quality control curve based on PBRTQC and grouping of the abnormal sample(s) in a first tab of a fourth display area 463 as shown in Fig. 13, and displays quality control product information and preferably an internal quality control diagram of the quality control product(s) in a second tab of the fourth display area as shown in Fig. 15.

Here, the controller 440 configured to receive a fourth interactive operation inputted by a user from the interactive device, may, for example, include: the controller configured to receive the fourth interactive operation of the user selecting the quality control product information and preferably the quality control diagram from the second tab of the fourth display area. In other words, the fourth interactive operation includes the user selecting the quality control product information from the second tab of the fourth display area.

In some embodiments, the controller 440 is configured to:
determine a target object of interest on the displayed target information management interface in response to a first interactive operation of the user; and
control the display device to switch back to displaying the real-time quality control interface on the display screen, or to display the real-time quality control interface on a same screen of the display screen, in response to a second interactive operation of the user, wherein the displayed real-time quality control interface 160 at least displays the quality control information based on PBRTQC that is related to the determined target object of interest indicated by the first interactive operation.

For example, as shown in Fig. 14, the controller 440 may be further configured to:
control the display device 420 to display the target information management interface 450 on the display screen 421, so that the target information management interface 450 also displays a virtual control 453 for invoking the real-time quality control interface 460;
receive the first interactive operation of the user selecting a target object of interest from the displayed target information management interface;
receive the second interactive operation of the user selecting the virtual control 453 from the displayed target information management interface; and
control the display device 420 to display the real-time quality control interface 460 on the display screen 421 in response to the second interactive operation, so that the real-time quality control interface at least displays real-time quality control information that is related to the target object of interest indicated by the first interactive operation;
preferably here, the virtual control 453 is a command button.

In some embodiments, the controller 440 may be further configured to activate the virtual control 453 in response to the first user instruction.

In some embodiments, the controller is further configured to receive a third interactive operation of the user from the real-time quality control interface 460, and delete or mark the quality control information based on PBRTQC for abnormal sample(s).

For example, when the user determines that the quality control information based on PBRTQC for the abnormal sample(s) causing an out-of-control alarm indicates false out-of-control through analysis, the user can mark this out-of-control alarm as false out-of-control on the real-time quality control interface 460. For example, in the embodiment shown in Fig. 13, the out-of-control alarm of the time period 2 is marked as false out-of-control. After marking, the abnormal state identification related to this out-of-control alarm is eliminated synchronously in the sample analysis system.

It may be understood that, deleting the quality control information based on PBRTQC for the abnormal sample(s) does not mean permanent deletion, but that these deleted quality control data are no longer used in the PBRTQC real-time quality control.

An embodiment of the present disclosure also relates to an information management method 500. As shown in Fig. 16, the method 500 includes:
S510, displaying a target information management interface on a display screen, so that the target information management interface displays information of a target object, the target object including at least one type of sample analyzer(s), sample(s), detection item(s) and quality control product(s), wherein the sample analyzer(s) is(are) configured to detect the samples for the detection item(s), so as to obtain detection results of the samples for the detection item(s), and the sample analyzer(s) is(are) quality-controlled for the detection item(s) through the quality control product(s);
S520, determining a target object of interest on the displayed target information management interface in response to a first interactive operation of a user, for example the first interactive operation being received from the displayed target information management interface; and
S530, displaying quality control information based on PBRTQC that is related to the determined target object of interest, on the display screen in response to a second interactive operation of the user, wherein the quality control information based on PBRTQC is generated based on the detection results of multiple of the samples for at least one of the detection item(s).

In some embodiments, the target information management interface is further configured for being capable of receiving a work instruction, so that at least one of the sample analyzer(s) can execute a corresponding operation based on the work instruction.

In some embodiments, each detection item that is quality-controlled through one of the quality control product(s) is associated with its respective quality control information based on PBRTQC.

In some embodiments, the step S530 includes: switching to displaying a real-time quality control interface, that is preferably independent of the target information management interface, on the display screen, or displaying a real-time quality control interface, that is preferably independent of the target information management interface, on a same screen of the display screen, in response to the second interactive operation of the user, so that the real-time quality control interface at least displays the quality control information based on PBRTQC that is related to the determined target object of interest.

In some embodiments, the target information management interface is a sample information management interface for displaying the plurality of samples and sample information thereof as well as corresponding detection item(s) of each sample and the detection results thereof, and the sample information management interface is configured for being capable of receiving a detection instruction, which is one type of the work instruction, so that at least one of the sample analyzer(s) can detect at least one of the samples for at least one of the detection item(s) based on the detection instruction.

Here, the target object of interest is a sample of interest and/or a detection item of interest. In other words, in the method 500, a sample of interest and/or a detection item of interest on the displayed sample information management interface is determined in response to the first interactive operation of the user, and it is switched to displaying the real-time quality control interface on the display screen, or the real-time quality control interface is displayed on a same screen of the display screen, in response to the second interactive operation of the user, so that the displayed real-time quality control interface at least displays the quality control information based on PBRTQC that is related to the determined sample of interest and/or detection item of interest.

In some embodiments, the second interactive operation may include the user triggering a virtual button(for example a virtual button) displayed on the target information management interface (for example the sample information management interface) for obtaining the quality control information based on PBRTQC, particularly for invoking the real-time quality control interface. Alternatively, the second interactive operation may include the user triggering a physical button for obtaining the quality control information based on PBRTQC, particularly for invoking the real-time quality control interface.

For example, in step S530, the second interactive operation may be received from an interactive device.

In some embodiments, the interactive device is set as a virtual control displayed on the target information management interface for obtaining the quality control information based on PBRTQC, particularly for invoking the real-time quality control interface; and
the step of receiving the second interactive operation from an interactive device, includes: receiving the second interactive operation of the user selecting the virtual control from the displayed target information management interface.

In some embodiments, the virtual control or the physical button is activated in response to the first interactive operation.

In some embodiments, the virtual control is a command button.

In some embodiments, at least the abnormal target object(s) is(are) displayed on the target information management interface, and the selected target object of interest is one of the abnormal target object(s). For example, in the case where the target information management interface is the sample information management interface, abnormal sample(s) and/or abnormal detection item(s) is(are) highlighted on the sample information management interface, wherein the sample of interest is one of the abnormal sample(s) and/or the determined detection item of interest is one of the abnormal detection item(s).

In some embodiments, in the case where the target information management interface is the sample information management interface, the step of displaying a target information management interface on a display screen, so that the target information management interface displays information of a target object may include:
displaying the sample information management interface on the display screen, so that the sample information management interface displays the plurality of samples and sample information thereof in a first display area, and displays the detection item(s) corresponding to the sample selected from the plurality of samples displayed on the first display area and to display the detection results thereof in a second display area; and, determining a target object of interest on the displayed target information management interface in response to a first interactive operation of a user, includes: determining a sample selected by the user from the plurality of samples displayed on the first display area, as the sample of interest, or determining a detection item selected by the user from the detection item(s) displayed on the second display area, as the detection item of interest.

In some embodiments, the step of displaying quality control information based on PBRTQC, particularly the real-time quality control interface on the display screen in response to a second interactive operation of the user may include one of the followings:
popping up a display frame that overlays the target information management interface (for example the sample information management interface) on the display screen, and displaying the quality control information based on PBRTQC, particularly the real-time quality control interface in the display frame in response to the second user instruction;
displaying the target information management interface (for example the sample information management interface) and the quality control information based on PBRTQC, particularly the real-time quality control interface on a same screen in different display areas in response to the second user instruction;
displaying the quality control information based on PBRTQC, particularly the real-time quality control interface on a tab on the display screen that is different from a tap where the target information management interface (for example the sample information management interface) is displayed, in response to the second user instruction; and
exiting from the target information management interface (for example the sample information management interface) and displaying the quality control information based on PBRTQC, particularly the real-time quality control interface in response to the second user instruction.

In some embodiments, the quality control information based on PBRTQC may include a quality control curve based on PBRTQC, and preferably the quality control information based on PBRTQC may further include abnormal sample(s) among the samples corresponding to the quality control curve based on PBRTQC, and grouping of the abnormal sample(s) as well as corresponding detection item(s) and the sample analyzer for detecting said samples, and further preferably the abnormal sample(s) may be marked on the quality control curve based on PBRTQC.

In some embodiments, the method 500 may further include: in response to a fourth interactive operation of the user,
switching back to displaying the target information management interface on the display screen (for example the sample information management interface); or
switching to displaying a quality control product information management interface on the display screen, wherein quality control product information management interface is configured to display quality control information, which is obtained by performing quality control on at least one of the sample analyzer(s) for at least one of the detection item(s) through the quality control product(s).

In some embodiments, in the case where the target information management interface is the sample information management interface, the method 500 may further include: before switching back to displaying the target information management interface on the display screen, determining an abnormal sample of interest from the abnormal sample(s) displayed on the real-time quality control interface and/or a detection item of interest corresponding to an abnormal sample in response to a fifth interactive operation of the user. Accordingly, switching back to displaying the target information management interface on the display screen in response to a fourth interactive operation of the user, includes: switching back to displaying the sample information management interface on the display screen in response to the fourth interactive operation of the user, so that the sample information management interface at least displays information related to the abnormal sample of interest and/or the detection item of interest corresponding to the abnormal sample.

Preferably, the fifth interactive operation may include: selecting the sample of interest from the quality control curve based on PBRTQC displayed on the real-time quality control interface, or selecting the sample of interest from the grouping displayed on the real-time quality control interface.

It should be understood here that, various embodiments described in connection with the sample analysis system 100 are also applicable to the method 500. Especially, the method 500 or its embodiment is performed by the above-described sample analysis system or its embodiment.

An embodiment of the present disclosure also provides an information management system, including a memory and a processor coupled to the memory, wherein the processor is configured to perform the method 500 according to any of the aforementioned embodiments based on instructions stored in the memory.

The memory may include, for example, a system memory, a fixed non-volatile storage medium, and the like. The system memory may store, for example, an operation system, an application program, a boot loader, and other programs.

The information management system may further comprise an I/O interface, a network interface, a storage interface, and the like. The I/O interface, the network interface and the storage interface therebetween as well as the memory and the processor therebetween may be connected, for example, via a bus. The I/O interface provides a connection interface for input and output devices such as a display, a mouse, a keyboard, and a touch screen. The network interface provides a connection interface for various networked devices. The storage interface provides a connection interface for an external storage device such as an SD card or a USB flash disk.

An embodiment of the present disclosure further provide a computer readable storage medium including computer program instructions that, when executed by a processor, implement the method 500 according to any of the above-described embodiments.

An embodiment of the present disclosure further provide a computer program product including a computer program that, when executed by a processor, implements the method 500 according to any of the above-described embodiments.

Hereto, various embodiments of the present disclosure have been described in detail. In order to avoid obscuring the concept of the present disclosure, some details commonly known in the art have not been described. From the above descriptions, those skilled in the art may fully understand how to implement the technical solutions disclosed here.

Those skilled in the art will appreciate that the embodiments of the present disclosure may be provided as methods, systems, or computer program products. Therefore, the present disclosure may take the form of an entirely hardware embodiment, an entirely software embodiment, or an embodiment combining software and hardware aspects. Moreover, the present disclosure may take the form of a computer program product implemented in one or more computer-usable non-transitory storage media (including but not limited to a disk memory, CD-ROM, an optical storage, and the like) containing computer usable program codes therein.

The present disclosure is described with reference to flow charts and/or block diagrams of methods, devices (systems), and computer program products according to the embodiments of the present disclosure. It should be understood that the functions specified in one or more flows in a flowchart and/or in one or more blocks in a block diagram may be implemented by computer program instructions. These computer program instructions may be provided to a processor of a general purpose computer, a special purpose computer, an embedded processing machine, or other programmable data processing devices to produce a machine, such that the instructions executed by the processor of the computer or other programmable data processing devices produce a device for realizing the functions specified in one or more flows of a flow chart and/or in one or more blocks in the block diagram.

These computer program instructions may also be stored in a computer readable memory that can guide a computer or other programmable data processing devices to operate in a particular manner, such that the instructions stored in the computer readable memory produce an article of manufacture including an instruction device, which implements functions specified in one or more flow s in a flow charts or in one or more blocks in a block diagram.

These computer program instructions may also be loaded onto a computer or other programmable data processing devices, so that a series of operational steps are executed on the computer or other programmable devices to produce a computer-implemented processing, such that the instructions executed on the computer or other programmable devices provide steps for realizing the functions specified in one or more flows of a flow chart and/or in one or more blocks in a block diagram.

Although some specific embodiments of the present disclosure have been described in detail by way of examples, those skilled in the art should understand that the above examples are only for an illustrative purpose, rather than limiting the scope of the present disclosure. Those skilled in the art should appreciate that modifications may be made to the above embodiments or equivalent replacements can be made to some technical features without departing from the scope and spirit of the present disclosure. The scope of the present disclosure is defined by the appended claims.

## Claims

1. A sample analysis system, comprising:
at least one sample analyzer configured to detect a plurality of samples for at least one detection item, so as to obtain detection results of the plurality of samples for the at least one detection item, wherein the at least one sample analyzer is quality-controlled for the at least one detection item through at least one quality control product;
a display device comprising a display screen for displaying a target information management interface and for displaying quality control information based on PBRTQC (Patient-Based Real-Time Quality Control), wherein the target information management interface is configured to display information of a target object, and the target object comprises at least one type of the sample analyzer(s), the samples, the detection item(s) and the quality control product(s), and the target information management interface is further configured for being capable of receiving a work instruction, so that at least one of the sample analyzer(s) can execute a corresponding operation based on the work instruction, wherein the quality control information based on PBRTQC is generated based on the detection results of multiple of the samples for at least one of the detection item(s), wherein each detection item that is quality-controlled through one of the quality control product(s) is associated with its respective quality control information based on PBRTQC; and
a controller communicatively connected with the display device and configured to:
control the display device to display the target information management interface on the display screen,
determine a target object of interest on the displayed target information management interface in response to a first interactive operation of a user, and
control the display device to display the quality control information based on PBRTQC on the display screen in response to a second interactive operation of the user, wherein the displayed quality control information based on PBRTQC at least comprises the quality control information based on PBRTQC that is related to the determined target object of interest.

2. The sample analysis system according to claim 1, wherein the controller configured to control the display device to display the quality control information based on PBRTQC on the display screen in response to a second interactive operation of the user, comprises: the controller configured to control the display device to switch to displaying a real-time quality control interface on the display screen, or control the display device to display a real-time quality control interface on a same screen of the display screen, in response to the second interactive operation of the user, wherein the displayed real-time quality control interface at least displays the quality control information based on PBRTQC that is related to the determined target object of interest;
preferably, the target information management interface is a sample information management interface, which is configured to display the plurality of samples and sample information thereof as well as corresponding detection item(s) of each sample and the detection results thereof, wherein the sample information management interface is further configured for being capable of receiving a detection instruction, which is one type of the work instruction, so that at least one of the sample analyzer(s) can detect at least one of the samples for at least one of the detection item(s) based on the detection instruction, wherein the target object of interest is a sample of interest and/or a detection item of interest;
preferably, the real-time quality control interface is independent of the target information management interface.

3. The sample analysis system according to claim 1 or 2, wherein the first interactive operation comprises one of the followings:
the user selecting the target object of interest from the target objects displayed on the target information management interface, preferably the user selecting the sample of interest from the samples displayed on the sample information management interface, and/or the user selecting the detection item of interest from the detection item(s) displayed on the sample information management interface;
the user selecting the target object of interest from the target objects displayed on the target information management interface by searching or screening, preferably the user selecting the sample of interest from the samples displayed on the sample information management interface, and/or the user selecting the detection item of interest from the detection item(s) displayed on the sample information management interface by searching or screening; and
the user selecting the target object of interest, preferably the sample of interest and/or the detection item of interest, by scanning a code.

4. The sample analysis system according to any of claims 1 to 3, wherein the second interactive operation comprises the user triggering a virtual button displayed on the target information management interface for obtaining the quality control information based on PBRTQC, particularly for invoking the real-time quality control interface; or wherein the second interactive operation comprises the user triggering a physical button for obtaining the quality control information based on PBRTQC, particularly for invoking the real-time quality control interface;
preferably the controller is further configured to activate the virtual button or the physical button in response to the first interactive operation.

5. The sample analysis system according to any of claims 1 to 4, wherein abnormal target object(s) is(are) highlighted on the target information management interface, wherein the target object of interest is one of the abnormal target object(s);
preferably the target information management interface is a sample information management interface, and abnormal sample(s) and/or abnormal detection item(s) is(are) highlighted on the sample information management interface, wherein the sample of interest is one of the abnormal sample(s) and/or the detection item of interest is one of the abnormal detection item(s).

6. The sample analysis system according to any of claims 1 to 5, wherein the target information management interface is a sample information management interface, which is configured to display the plurality of samples and sample information thereof as well as corresponding detection item(s) of each sample and the detection results thereof, wherein the sample information management interface is further configured for being capable of receiving a detection instruction, which is one type of the work instruction, so that at least one of the sample analyzer(s) can detect at least one of the samples for at least one of the detection item(s) based on the detection instruction, wherein the target object of interest is a sample of interest and/or a detection item of interest;
wherein the sample information management interface comprises a first display area and a second display area, wherein the first display area is configured to display the plurality of samples and sample information thereof, and the second display area is configured to display the detection item(s) corresponding to the sample selected from the plurality of samples displayed on the first display area and to display the detection results thereof; and
wherein the controller configured to determine a target object of interest on the displayed target information management interface in response to a first interactive operation of a user, comprises: the controller configured to determine a sample selected by the user from the plurality of samples displayed on the first display area, as the sample of interest, or to determine a detection item selected by the user from the detection item(s) displayed on the second display area, as the detection item of interest.

7. The sample analysis system according to any of claims 1 to 6, wherein the controller is further configured to control the display device to display the quality control information based on PBRTQC, particularly the real-time quality control interface, on the display screen by one of the following methods:
popping up a display frame that overlays the target information management interface on the display screen, and displaying the quality control information based on PBRTQC, particularly the real-time quality control interface, in the display frame;
displaying the quality control information based on PBRTQC, particularly the real-time quality control interface, on a tab that is different from a tab where the target information management interface is displayed; and
exiting from the target information management interface and displaying the quality control information based on PBRTQC, particularly the real-time quality control interface.

8. The sample analysis system according to any of claims 1 to 7, wherein the quality control information based on PBRTQC comprises a quality control curve based on PBRTQC, and preferably the quality control information based on PBRTQC further comprises abnormal sample(s) among the samples corresponding to the quality control curve based on PBRTQC, and grouping of the abnormal sample(s) as well as corresponding detection item(s) and the sample analyzer for detecting said samples, and further preferably the abnormal sample(s) is(are) marked on the quality control curve based on PBRTQC;
preferably the controller is further configured to delete or mark the quality control information based on PBRTQC for the abnormal sample(s) in response to a third interactive operation of the user.

9. The sample analysis system according to any of claims 1 to 8, wherein the controller is further configured to: in response to a fourth interactive operation of the user,
control the display device to switch back to displaying the target information management interface on the display screen; or
control the display device to switch to displaying a quality control product information management interface on the display screen, wherein quality control product information management interface is configured to display quality control information, which is obtained by performing quality control on at least one of the sample analyzer(s) for at least one of the detection item(s) through the quality control product(s).

10. The sample analysis system according to claim 9, wherein the target information management interface is a sample information management interface, and the controller is further configured to: before the controller controls the display device to switch back to displaying the target information management interface on the display screen, determine an abnormal sample of interest from the displayed abnormal sample(s) and/or a detection item of interest corresponding to an abnormal sample in response to a fifth interactive operation of the user; and
the controller further configured to control the display device to switch back to displaying the target information management interface on the display screen in response to a fourth interactive operation of the user, comprises: the controller further configured to control the display device to switch back to displaying the sample information management interface on the display screen in response to the fourth interactive operation of the user, so that the sample information management interface at least displays information related to the abnormal sample of interest and/or the detection item of interest corresponding to the abnormal sample;
preferably the quality control information based on PBRTQC comprises a quality control curve based on PBRTQC, and the quality control information based on PBRTQC further comprises abnormal sample(s) among the samples corresponding to the quality control curve based on PBRTQC, and grouping of the abnormal sample(s) as well as corresponding detection item(s); and
the fifth interactive operation comprises: selecting the sample of interest from the displayed quality control curve based on PBRTQC, or selecting the sample of interest from the displayed grouping.

11. The sample analysis system according to any of claims 1 to 10, wherein the display device and the controller are integrated in a computer system that is directly or indirectly communicatively connected with the sample analyzer(s); or
the display device and the controller are a display device and a controller of one of the sample analyzer(s).

12. An information management system, comprising:
an information management module configured to generate a target information management interface for displaying information of a target object, wherein the target object comprises at least one type of sample analyzer(s), samples, detection item(s) and quality control product(s), the sample analyzer(s) is(are) configured to detect the samples for the detection item(s), so as to obtain detection results of the samples for the detection item(s), and the sample analyzer(s) is(are) quality-controlled for the detection item(s) through the quality control product(s), wherein the target information management interface is further configured for being capable of receiving a work instruction, so that at least one of the sample analyzer(s) can execute a corresponding operation based on the work instruction;
a real-time quality control module configured to generate quality control information based on PBRTQC, wherein the quality control information based on PBRTQC is generated based on the detection results of multiple of the samples for at least one of the detection item(s), wherein each detection item that is quality-controlled through one of the quality control product(s) is associated with its respective quality control information based on PBRTQC; and
a control module configured to:
control the information management module to generate the target information management interface and output the target information management interface to a display screen, so as to display the target information management interface on the display screen,
determine a target object of interest on the displayed target information management interface in response to a first interactive operation of a user, and
control the real-time quality control module to generate the quality control information based on PBRTQC and output the quality control information based on PBRTQC to the display screen in response to a second interactive operation of the user, so as to display the quality control information based on PBRTQC on the display screen, wherein the displayed the quality control information based on PBRTQC at least comprises the quality control information based on PBRTQC that is related to the determined target object of interest.

13. The information management system according to claim 12, wherein the real-time quality control module is configured to generate a real-time quality control interface for displaying the quality control information based on PBRTQC;
wherein the control module configured to control the real-time quality control module to generate the quality control information based on PBRTQC and output the quality control information based on PBRTQC to the display screen in response to a second interactive operation of the user, comprises: the control module configured to control the real-time quality control module to generate the real-time quality control interface and output the real-time quality control interface to the display screen in response to the second interactive operation of the user, so as to switch to displaying the real-time quality control interface on the display screen, or to display the real-time quality control interface on a same screen of the display screen, wherein the displayed real-time quality control interface at least displays the quality control information based on PBRTQC that is related to the determined target object of interest;
preferably, the target information management interface is a sample information management interface, which is configured to display the plurality of samples and sample information thereof as well as corresponding detection item(s) of each sample and the detection results thereof, wherein the sample information management interface is further configured for being capable of receiving a detection instruction, which is one type of the work instruction, so that at least one of the sample analyzer(s) can detect at least of the samples for at least of the detection item(s) based on the detection instruction, wherein the target object of interest is a sample of interest and/or a detection item of interest;
preferably, the real-time quality control interface is independent of the target information management interface.

14. The information management system according to claim 12 or 13, wherein first interactive operation comprises one of the followings:
the user selecting the target object of interest from the target objects displayed on the target information management interface, preferably the user selecting the sample of interest from the samples displayed on the sample information management interface, and/or the user selecting the detection item of interest from the detection item(s) displayed on the sample information management interface;
the user selecting the target object of interest from the target objects displayed on the target information management interface by searching or screening, preferably the user selecting the sample of interest from the samples displayed on the sample information management interface, and/or the user selecting the detection item of interest from the detection item(s) displayed on the sample information management interface by searching or screening; and
the user selecting the target object of interest, preferably the sample of interest and/or the detection item of interest, by scanning a code;
and/or
wherein the second interactive operation comprises the user triggering a virtual button displayed on the target information management interface for obtaining the quality control information based on PBRTQC, particularly for invoking the real-time quality control interface; or wherein the second interactive operation comprises the user triggering a physical button for obtaining the quality control information based on PBRTQC, particularly for invoking the real-time quality control interface;
preferably the control module is further configured to activate the virtual button or the physical button in response to the first interactive operation.

15. An information management method, comprising:
displaying a target information management interface on a display screen, so that the target information management interface displays information of a target object, the target object comprising at least one type of sample analyzer(s), samples, detection item(s) and quality control product(s), wherein the sample analyzer(s) is(are) configured to detect the samples for the detection item(s), so as to obtain detection results of the samples for the detection item(s), and the sample analyzer(s) is(are) quality-controlled for the detection item(s) through the quality control product(s), wherein the target information management interface is further configured for being capable of receiving a work instruction, so that at least one of the sample analyzer(s) can execute a corresponding operation based on the work instruction, wherein each detection item that is quality-controlled through one of the quality control product(s) is associated with its respective quality control information based on PBRTQC;
determining a target object of interest on the displayed target information management interface in response to a first interactive operation of a user, and
displaying quality control information based on PBRTQC related to the determined target object of interest on the display screen, in response to a second interactive operation of the user, wherein the displayed control information based on PBRTQC at least comprises the quality control information based on PBRTQC that is related to the determined target object of interest.
